# EUROPÄISCHE PATENTSCHRIFT

(11) **EP 4 164 549 B1**
(45) Veröffentlichungstag und Bekanntmachung des Hinweises auf die Patenterteilung: **20.12.2023**
(21) Anmeldenummer: 21731959.9
(22) Anmeldetag: 07.06.2021
(51) Int. Cl.: A61F 2/16

(54) **INJEKTORANORDNUNG FÜR EIN EINFÜHREN EINER INTRAOKULARLINSE UND INJEKTOR**
INJECTOR ASSEMBLY FOR INSERTING AN INTRAOCULAR LENS AND INJECTOR
ENSEMBLE INJECTEUR POUR L'INSERTION D'UNE LENTILLE INTRAOCULAIRE ET INJECTEUR

(30) Priorität: 10.06.2020 DE 102020115489
(43) Veröffentlichungstag der Anmeldung: 19.04.2023
(73) Patentinhaber: Carl Zeiss Meditec AG, 07745 Jena (DE)
(72) Erfinder: DAMM, Niklas, 10409 Berlin (DE); JASTRAM, Jakob, 12103 Berlin (DE); RINMAN, Alfred, 20357 Hamburg (DE)
(74) Vertreter: PATERIS Patentanwälte PartmbB
(86) Internationale Anmeldenummer: PCT/EP2021/065173
(87) Internationale Veröffentlichungsnummer: WO 2021/249944

(56) Entgegenhaltungen:
- WO-A1-2012/155887
- WO-A1-2013/038688
- US-A1- 2016 331 587

## Beschreibung

Die Erfindung betrifft eine Injektoranordnung für ein Einführen einer Intraokularlinse in den Kapselsack eines Auges und einen ophthalmochirurgischen Injektor mit der Injektoranordnung.

Bei einer Kataraktbehandlung eines Auges wird herkömmlich nur ein kleiner Schnitt in die Hornhaut des Auges eingebracht, der so groß ist, dass eine Kanüle eines Injektors durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Kanüle passt. Die Kanüle wird durch den Schnitt in den Kapselsack eingeführt und die gefaltete Intraokularlinse wird mittels des Injektors durch die Kanüle in den Kapselsack geschoben, in dem die Intraokularlinse sich entfaltet und somit die ursprüngliche Linse ersetzt. Während die Intraokularlinse gefaltet wird oder während die gefaltete Intraokularlinse in den Kapselsack geschoben wird, kann es zu Fehlern kommen. Die Fehler können beispielsweise dazu führen, dass sich die Intraokularlinse nicht vollständig in dem Kapselsack entfaltet oder dass Intraokularlinse sogar beschädigt wird.

WO 2012/155887 A1 betrifft ein Injektorsystem zum Implantieren einer Intraokularlinse in ein Auge sowie ein Magazin für das Injektorsystem. US 2016/331587 A1 offenbart einen Injektor für eine Intraokularlinse.

Aufgabe der Erfindung ist es daher, eine Injektoranordnung zu schaffen, mit der eine Wahrscheinlichkeit gering ist, dass Fehler bei einer Benutzung der Injektoranordnung auftreten.

Die Aufgabe wird durch eine Injektoranordnung gemäß dem unabhängigen Anspruch 1 gelöst. Vorteilhafte Weiterbildungen der Erfindung sind Gegenstand der Unteransprüche.

Die erfindungsgemäße Injektoranordnung weist auf: eine Faltkammer, welche eingerichtet ist, eine Intraokularlinse, die einen Optikkörper und zwei Haptiken aufweist, aufzunehmen, einen Halter, einen Faltkeil, der an dem Halter angebracht ist und eingerichtet ist, via eine Faltkammeröffnung in die Faltkammer eingebracht zu werden und dadurch die Intraokularlinse zu falten, sowie einen ersten Gleiter und einen zweiten Gleiter, die jeweils eingerichtet sind, durch ein Verschieben des jeweiligen Gleiters aus einer Ausgangsposition in eine Endposition eine der zwei Haptiken auf den Optikkörper zu verschieben, wobei der Halter eingerichtet ist, durch ein Bewegen des Halters zu der Faltkammer hin den ersten Gleiter und/oder den zweiten Gleiter aus der Ausgangsposition in die Endposition anzutreiben.

Indem die zwei Haptiken mittels der Gleiter auf den Optikkörper angeordnet werden, werden die Haptiken, wenn die Intraokularlinse von dem Faltkeil gefaltet wird, innerhalb des Optikkörpers angeordnet. Dadurch kann ein fehlerarmes und reproduzierbares Einbringen der Intraokularlinse in den Kapselsack eines Auges und ein fehlerarmes und reproduzierbares Entfalten der Intraokularlinse in dem Kapselsack erreicht werden. Indem der Halter den ersten Gleiter und/oder den zweiten Gleiter in die Endposition antreibt, kann vermieden werden, dass der Faltkeil in die Faltkammer eingebracht wird, bevor der erste Gleiter und/oder der zweite Gleiter in die Endposition gebracht wurde und somit die zu dem mindestens einen Gleiter zugehörige Haptik auf den Optikkörper geschoben wurde. Dadurch ist Injektoranordnung besonders fehlerarm. Ganz besonders fehlerarm ist die Injektoranordnung, wenn der Halter eingerichtet ist, durch das Bewegen des Halters zu der Faltkammer hin sowohl den ersten Gleiter als auch den zweiten Gleiter aus der Ausgangsposition in die Endposition anzutreiben.

Die Injektoranordnung weist bevorzugt einen Injektorkörper, in dessen Innerem die Faltkammer angeordnet ist, und ein Gelenk auf, mittels dem der Halter verschwenkbar an dem Injektorkörper angebracht ist. Damit ist der Halter verschwenkbar relativ zu dem Injektorkörper und durch ein Verschwenken des Halters um das Gelenk herum in Richtung zu dem Optikkörper hin wird der Faltkeil in die Faltkammer eingebracht.

Es ist bevorzugt, dass die Injektoranordnung einen Schieber aufweist, der an dem ersten Gleiter befestigt ist und sich ausgehend von dem ersten Gleiter in Richtung zu dem Gelenk erstreckt, wobei der Halter eingerichtet ist, mittels eines Verschwenkens des Halters um das Gelenk herum in Richtung zu der Faltkammer hin, den Schieber anzutreiben, der dadurch den ersten Gleiter in die Endposition verschiebt. Dabei ist denkbar, dass bei dem Verschwenken der Halter oder ein an dem Halter befestigtes Bauteil den Schieber kontaktiert und somit den Schieber antreibt.

Alternativ ist es bevorzugt, dass die Injektoranordnung einen an dem Halter befestigten ersten Vorsprung mit einer ersten Gleitfläche aufweist, die eingerichtet ist, wenn der Halter um das Gelenk herum in Richtung zu der Faltkammer hin verschwenkt wird, an dem ersten Gleiter entlang zu gleiten und dadurch den ersten Gleiter in die Endposition zu verschieben. Dabei ist es besonders bevorzugt, dass die Injektoranordnung einen an dem Halter befestigten zweiten Vorsprung mit einer zweiten Gleitfläche aufweist, die eingerichtet ist, wenn der Halter um das Gelenk herum in Richtung zu der Faltkammer hin verschwenkt wird, an dem zweiten Gleiter entlang zu gleiten und dadurch den zweiten Gleiter in die Endposition zu verschieben.

Es ist bevorzugt, dass der erste Gleiter einen ersten Gleitervorsprung aufweist, der von einer dem zweiten Gleiter abgewandten Seite des ersten Gleiters vorsteht und an dem die erste Gleitfläche eingerichtet ist zu gleiten, und/oder dass der zweite Gleiter einen zweiten Gleitervorsprung aufweist, der von einer dem ersten Gleiter abgewandten Seite des zweiten Gleiters vorsteht und an dem die zweite Gleitfläche eingerichtet ist zu gleiten. Die erste Gleitfläche und/oder die zweite Gleitfläche sind bevorzugt gekrümmt ausgeführt.

Es ist bevorzugt, dass die Injektoranordnung ein Rad aufweist, das eingerichtet ist, durch eine Rotation des Rads den ersten Gleiter von der Ausgangsposition in die Endposition zu verschieben, und der Halter eingerichtet ist, mittels eines Verschwenkens des Halters um das Gelenk herum in Richtung zu der Faltkammer hin das Rad in die Rotation zu versetzen. Das Rad ist besonders bevorzugt eingerichtet, durch die Rotation den zweiten Gleiter von der Ausgangsposition in die Endposition zu verschieben.

Es ist bevorzugt, dass die Injektoranordnung eine erste Stange aufweist, mittels der das Rad mit dem ersten Gleiter gekuppelt ist, um den ersten Gleiter anzutreiben, und/oder eine zweite Stange aufweist, mittels der das Rad mit dem zweiten Gleiter gekuppelt ist, um den zweiten Gleiter anzutreiben.

Es ist bevorzugt, dass das Gelenk eine Achse aufweist, die bei dem Verschwenken des Halters rotiert und an der ein erstes Zahnrad befestigt ist, und das Rad ein zweites Zahnrad ist, deren Zähne mit den Zähnen des ersten Zahnrads in Eingriff stehen. Dadurch, dass das erste Zahnrad an der Achse befestigt ist, rotiert das erste Zahnrad bei dem Verschwenken des Halters gemeinsam mit der Achse. Durch den Eingriff der Zähne des ersten Zahnrads und der Zähne des zweiten Zahnrads treibt das erste Zahnrad das zweite Zahnrad an. Dabei ist denkbar, dass die Rotationsachse des ersten Zahnrads im Wesentlichen senkrecht zu der Rotationsachse des zweiten Zahnrads angeordnet ist. Zudem kann die Rotationsachse des ersten Zahnrads mit der Rotationsachse der Achse zusammenfallen.

Die Injektoranordnung weist bevorzugt die Intraokularlinse auf. Besonders bevorzugt ist die Intraokularlinse in der Faltkammer angeordnet.

Der erfindungsgemäße ophthalmochirurgischer Injektor weist die Injektoranordnung auf.

Im Folgenden wird anhand der beigefügten schematischen Zeichnung die Erfindung näher erläutert.
Figur 1 zeigt eine perspektivische Ansicht einer ersten Ausführungsform der erfindungsgemäßen Injektoranordnung mit einem ersten Gleiter, der sowohl in der Ausgangsposition als auch in der Endposition dargestellt ist.
Figur 2 zeigt eine Seitenansicht der ersten Ausführungsform mit dem ersten Gleiter, der in der Endposition dargestellt ist.
Figur 3 zeigt eine Seitenansicht einer zweiten Ausführungsform der erfindungsgemäßen Injektoranordnung mit einem ersten Gleiter, der in einer Ausgangsposition dargestellt ist.
Figur 4 zeigt eine Seitenansicht der zweiten Ausführungsform mit dem ersten Gleiter, der in einer Endposition dargestellt ist.
Figur 5 zeigt eine Seitenansicht einer dritten Ausführungsform der erfindungsgemäßen Injektoranordnung mit einem ersten Gleiter und einem zweiten Gleiter, die in einer Ausgangsposition dargestellt sind.
Figur 6 zeigt eine Draufsicht einer vierten Ausführungsform der erfindungsgemäßen Injektoranordnung mit einem ersten Gleiter und einem zweiten Gleiter, die in einer Ausgangsposition dargestellt sind.

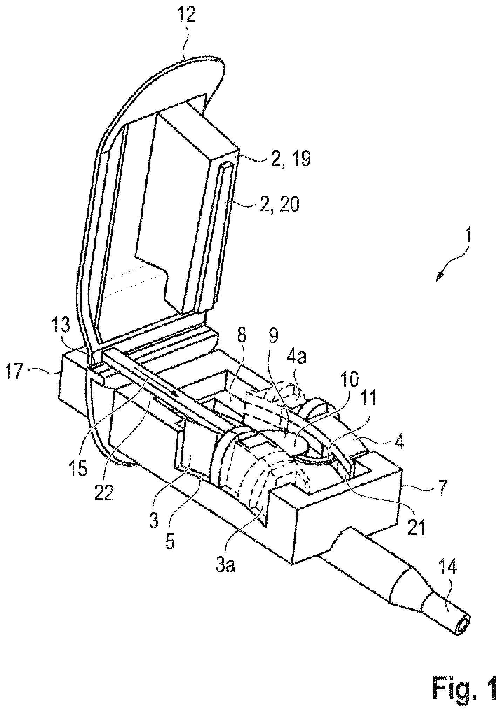

Wie es aus Figuren 1 bis 6 ersichtlich ist, weist eine Injektoranordnung 1 auf: eine Faltkammer 8, welche eingerichtet ist, eine Intraokularlinse 9, die einen Optikkörper 10 und zwei Haptiken 11 aufweist, aufzunehmen, einen Halter 12, einen Faltkeil 2, der an dem Halter 12 angebracht ist und eingerichtet ist, via eine Faltkammeröffnung 18 in die Faltkammer 8 eingebracht zu werden und dadurch die Intraokularlinse 9 zu falten, sowie einen ersten Gleiter 3 und einen zweiten Gleiter 4, die jeweils eingerichtet sind, durch ein Verschieben des jeweiligen Gleiters 3, 4 aus einer Ausgangsposition in eine Endposition eine der zwei Haptiken 11 auf den Optikkörper 10 zu verschieben, wobei der Halter 12 eingerichtet ist, durch ein Bewegen des Halters 12 zu der Faltkammer 8 hin den ersten Gleiter 3 und/oder den zweiten Gleiter 4 aus der Ausgangsposition in die Endposition anzutreiben.

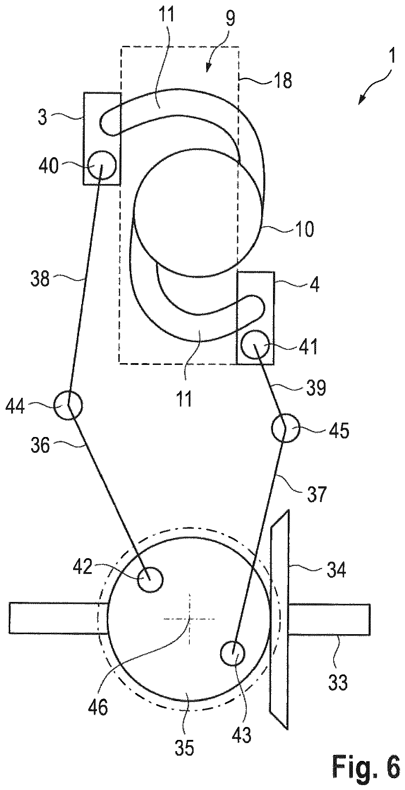

Die Injektoranordnung 1 kann die Intraokularlinse 9 aufweisen. Dabei ist denkbar, dass die Intraokularlinse 1 in der Faltkammer 8 angeordnet ist. Figuren 1 und 6 zeigen, dass die zwei Haptiken 11 jeweils C-förmig ausgebildet sein können.

Figuren 1 und 6 zeigen, dass der erste Gleiter 3 und der zweite Gleiter 4 an einander abgewandten Enden der Faltkammer 8 angeordnet sein können. Insbesondere können der erste Gleiter 3 und der zweite Gleiter 4 an einander abgewandten Enden der Faltkammeröffnung 18 angeordnet sein. Die Enden können in einer Richtung senkrecht zu einer Verschieberichtung, in der die Intraokularlinse 9 von der Faltkammer 8 zu einer Kanüle 14 der Injektoranordnung 1 zu verschieben ist, abgewandt voneinander angeordnet sein. Zudem ist erkennbar, dass die Injektoranordnung 1 eine erste Gleiterbahn 5, auf der der erste Gleiter 3 gleitend gelagert ist, und eine zweite Gleiterbahn (nicht dargestellt) aufweisen kann, auf der der zweite Gleiter 4 gleitend gelagert ist. Die erste Gleiterbahn 5 und die zweite Gleiterbahn können gekrümmt ausgeführt sein, so dass die Gleiter 3, 4, wenn sie von der Ausgangsposition in die Endposition verschoben werden, einen gekrümmten Weg zurücklegen. Dadurch kann beim Verschieben der Haptiken 11 eine Kollision der Haptiken 11 mit dem Umfangsrand des Optikkörpers 10 vermieden werden. Aus Figur 1 ist ersichtlich, dass die Gleiter 3, 4 jeweils einen Gleiterzapfen 21 aufweisen können, der in Richtung zu der Introkularlinse 9 hin von dem Gleiter 3, 4 absteht. Jeder der Gleiterzapfen 21 ist eingerichtet, jeweils eine der zwei Haptiken 11 zu greifen und auf den Optikkörper 10 zu verschieben, wenn der zu dem Gleiterzapfen 21 zugehörige Gleiter 3, 4 von der Ausgangsposition in die Endposition verschoben wird. Die Gleiterzapfen 21 können von einer Seite des Gleiters 3, 4 abstehen, die dem außerhalb der Faltkammer 8 angeordneten Faltkeil 2 abgewandt angeordnet ist. Dadurch wird erreicht, dass die Haptiken 11 an diejenige Seite des Optikkörpers 10 verschoben werden, die dem Faltkeil 2 zugewandt ist. Dadurch kann erreicht werden, dass die Haptiken 11 innerhalb des Optikkörpers 10 angeordnet werden, wenn der Faltkeil 2 die Intraokularlinse 9 faltet.

Figuren 1 bis 4 zeigen, dass die Injektoranordnung 1 einen Injektorkörper 7, in dessen Innerem die Faltkammer 8 angeordnet ist, und ein Gelenk 13 aufweisen kann, mittels dem der Halter 12 verschwenkbar an dem Injektorkörper 7 angebracht ist. Durch ein Verschwenken des Halters 12 um das Gelenk 13 herum in Richtung zu dem Injektorkörper 7 hin kann der Faltkeil 2 in die Faltkammer 8 eingebracht werden und dadurch die Intraokularlinse 9 gefaltet werden.

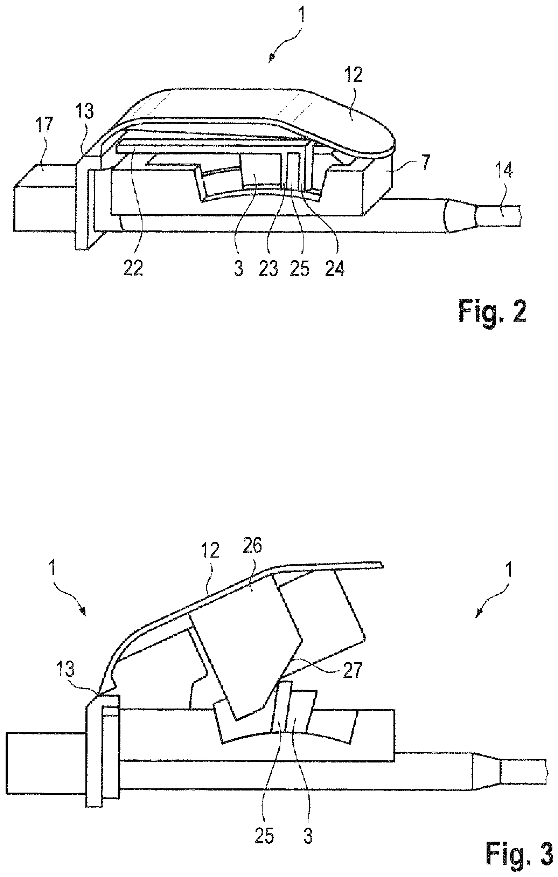

Beispielsweise Figuren 1 und 2 zeigen, dass die Injektoranordnung 1 einen Adapter 17 aufweisen kann, der eingerichtet ist, mit einem Kolben gekuppelt zu werden, in dem ein Stempel längsverschiebbar gelagert ist. Es ist denkbar, dass ein ophthalmochirurgischer Injektor geschaffen wird, indem die Injektoranordnung 1 mittels des Adapters 17 mit dem Kolben gekuppelt wird. Der ophthalmochirurgische Injektor weist somit die Injektoranordnung 1, den Kolben und den Stempel auf. Der Stempel kann eingerichtet sein, via den Adapter 17 in die Faltkammer 8 eingeführt zu werden und die von dem Faltkeil 2 gefaltete Intraokularlinse 9 aus der Faltkammer 8 heraus in eine Kanüle 14 der Injektoranordnung 1 zu schieben und von der Kanüle 14 aus der Injektoranordnung 1 heraus zu schieben.

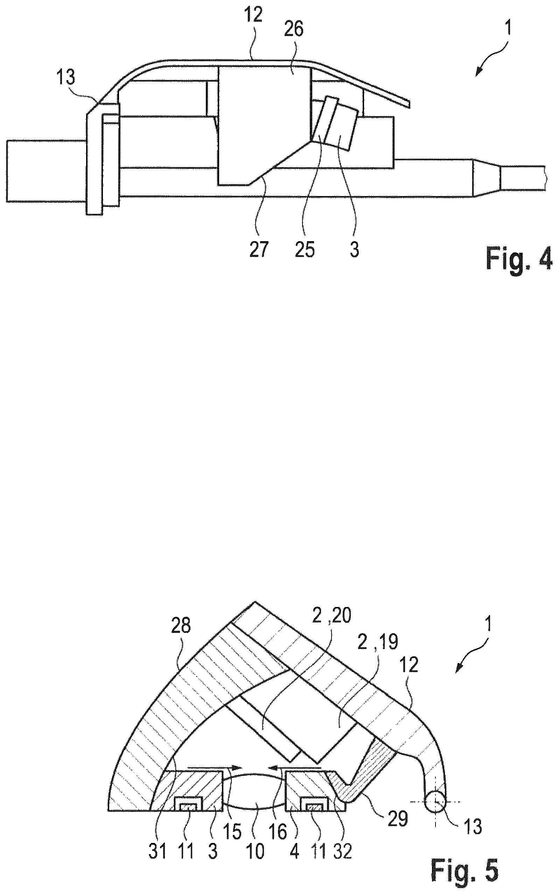

Figuren 1 und 5 zeigen, dass der Faltkeil 2 ein feststehendes Faltkeilteil 19, das an dem Halter 12 befestigt ist, und ein bewegliches Faltkeilteil 20 aufweist, das beweglich an dem feststehenden Faltkeilteil 19 angebracht ist und von dem feststehenden Faltkeilteil 19 vorstehen kann. Das feststehende Faltkeilteil 19 kann einen Schlitz aufweisen, in dem das bewegliche Faltkeilteil 20 angeordnet ist. Durch die Anordnung mit dem feststehenden Faltkeilteil 19 und dem beweglichen Faltkeilteil 20 kann unterbunden werden, dass die Intraokularlinse 9 seitlich in Richtung zu dem Halter 12 hin wegrutscht und verklemmt.

Figuren 1 und 2 zeigen eine erste Ausführungsform für die Injektoranordnung 1. Bei der ersten Ausführungsform weist die Injektoranordnung 1 einen Schieber 22 auf, der an dem ersten Gleiter 3 befestigt ist und sich ausgehend von dem ersten Gleiter 3 in Richtung zu dem Gelenk 13 erstreckt, wobei der Halter 12 eingerichtet ist, mittels eines Verschwenkens des Halters 12 um das Gelenk 13 herum in Richtung zu der Faltkammer 8 hin, den Schieber 22 anzutreiben, der dadurch den ersten Gleiter 3 in die Endposition verschiebt. In Figur 1 ist der erste Gleiter sowohl in seiner mit dem Bezugszeichen 3 bezeichneten Ausgangsposition als auch in seiner mit dem Bezugszeichen 3a bezeichneten Endposition dargestellt (mit gestrichelter Linie gezeichnet). Der Faltkeil 2 ist außerhalb der Faltkammer 8 angeordnet. In Figur 2 ist dargestellt, dass der Halter 12 in Richtung zu der Faltkammer 8 hin verschwenkt wurde und somit der Schieber 22 in einer Richtung weg von dem Gelenk 13 verschoben wurde, wodurch der erste Gleiter 3 in die Endposition gebracht wurde. Der Schieber 22 kann bei dem Verschwenken des Halters 12 in Kontakt mit dem Halter 12 oder mit einem an dem Halter 12 befestigten Bauteil treten, ohne jedoch an dem Halter 12 oder an dem Bauteil befestigt zu sein.

Um den Schieber 22 an dem ersten Gleiter 3 zu befestigen, kann der erste Gleiter 3 einen Gleitervorsprung 25 aufweisen, der von einer dem zweiten Gleiter 4 abgewandten Seite des ersten Gleiters 3 vorsteht. Der Schieber 22 kann einen ersten Schieberzapfen 23 und einen zweiten Schieberzapfen 24 aufweisen, zwischen denen der Gleitervorsprung 25 eingeklemmt ist.

Figuren 3 und 4 zeigen eine zweite Ausführungsform für die Injektoranordnung 1. Bei der zweiten Ausführungsform weist die Injektoranordnung 1 einen an dem Halter 12 befestigten ersten Vorsprung 26 mit einer ersten Gleitfläche 27 auf, die eingerichtet ist, wenn der Halter 12 um das Gelenk 13 herum in Richtung zu der Faltkammer 8 hin verschwenkt wird, an dem ersten Gleiter 3 entlang zu gleiten und dadurch den ersten Gleiter 3 in die Endposition zu verschieben. Der erste Gleiter 3 weist einen ersten Gleitervorsprung 25 auf, der von einer dem zweiten Gleiter 4 abgewandten Seite des ersten Gleiters 3 vorsteht und an dem die erste Gleitfläche 27 eingerichtet ist, zu gleiten. Die erste Gleitfläche 27 hat die Form einer Ebene. Alternativ ist denkbar, dass die erste Gleitfläche 27 gekrümmt ausgeführt ist. Dabei ist denkbar, dass die erste Gleitfläche 27 konvex ausgeführt ist in dem Fall, dass die erste Gleitfläche 27 eingerichtet ist, den ersten Gleiter 3 weg von dem Gelenk 13 zu verschieben, und konkav eingerichtet ist in dem Fall, dass die erste Gleitfläche 27 eingerichtet ist, den ersten Gleiter 3 hin zu dem Gelenk 13 zu verschieben. Dadurch kann die erste Gleiterfläche 27 leicht an dem ersten Gleiter 3 gleiten.

Zudem ist denkbar, dass die Injektoranordnung 1 einen an dem Halter 12 befestigten zweiten Vorsprung mit einer zweiten Gleitfläche aufweist, die eingerichtet ist, wenn der Halter 12 um das Gelenk 13 herum in Richtung zu der Faltkammer 8 hin verschwenkt wird, an dem zweiten Gleiter 4 entlang zu gleiten und dadurch den zweiten Gleiter 4 in die Endposition zu verschieben. Dabei weist der zweite Gleiter 4 einen zweiten Gleitervorsprung auf, der von einer dem ersten Gleiter 3 abgewandten Seite des zweiten Gleiters 4 vorsteht und an dem die zweite Gleitfläche eingerichtet ist zu gleiten. Die zweite Gleitfläche kann die Form einer Ebene haben oder gekrümmt ausgeführt sein. Wenn die zweite Gleitfläche gekrümmt ausgeführt ist, ist es denkbar, dass die erste Gleitfläche 27 konvex ausgeführt ist in dem Fall, dass die zweite Gleitfläche eingerichtet ist, den zweiten Gleiter 4 weg von dem Gelenk 13 zu verschieben, und konkav eingerichtet ist in dem Fall, dass die zweite Gleitfläche eingerichtet ist, den zweiten Gleiter 4 hin zu dem Gelenk 13 zu verschieben. Dadurch kann die zweite Gleiterfläche leicht an dem zweiten Gleiter 4 gleiten.

Figur 5 zeigt eine dritte Ausführungsform für die Injektoranordnung 1. Bei der dritten Ausführungsform weist die Injektoranordnung einen an dem Halter 12 befestigten ersten Vorsprung 28 mit einer ersten Gleitfläche 31 auf, die eingerichtet ist, wenn der Halter 12 um das Gelenk 13 herum in Richtung zu der Faltkammer 8 hin verschwenkt wird, an dem ersten Gleiter 3 entlang zu gleiten und dadurch den ersten Gleiter 3 in die Endposition zu verschieben. Die erste Gleitfläche 31 kann eingerichtet sein, bei dem Verschwenken des Halters 12 eine dem Gelenk 13 abgewandte Seite des ersten Gleiters 3 zu kontaktieren und dadurch den ersten Gleiter 3 in einer ersten Bewegungsrichtung 15 in Richtung zu dem Gelenk 13 hin zu verschieben. Zudem ist es denkbar, dass die erste Gleitfläche 31 gekrümmt und insbesondere konkav ausgeführt ist.

Zudem ist es denkbar, dass die Injektoranordnung 1 einen an dem Halter 12 befestigten zweiten Vorsprung 29 mit einer zweiten Gleitfläche 32 aufweist, die eingerichtet ist, wenn der Halter 12 um das Gelenk 13 herum in Richtung zu der Faltkammer 8 hin verschwenkt wird, an dem zweiten Gleiter 4 entlang zu gleiten und dadurch den zweiten Gleiter 4 in die Endposition zu verschieben. Die zweite Gleitfläche 32 kann eingerichtet sein, bei dem Verschwenken des Halters 12 eine dem Gelenk 13 zugewandte Seite des zweiten Gleiters 4 zu kontaktieren und dadurch den zweiten Gleiter 4 in einer zweiten Bewegungsrichtung 16 von dem Gelenk 13 weg zu verschieben. Zudem ist es denkbar, dass die zweite Gleitfläche 32 gekrümmt und insbesondere konvex ausgeführt ist.

Figur 6 zeigt eine vierte Ausführungsform für die Injektoranordnung 1. Bei der vierten Ausführungsform weist die Injektoranordnung 1 ein Rad 35 auf, das eingerichtet ist, durch eine Rotation des Rads 35 den ersten Gleiter 3 von der Ausgangsposition in die Endposition zu verschieben, und der Halter 12 eingerichtet ist, mittels eines Verschwenkens des Halters 12 um das Gelenk 13 herum in Richtung zu der Faltkammer 8 hin das Rad 35 in die Rotation zu versetzen. Zudem kann das Rad eingerichtet sein, durch die Rotation den zweiten Gleiter 4 von der Ausgangsposition in die Endposition zu verschieben.

Dabei ist denkbar, dass die Injektoranordnung 1 eine erste Stange 36 aufweist, mittels der das Rad 35 mit dem ersten Gleiter 3 gekuppelt ist, um den ersten Gleiter 3 anzutreiben, und eine zweite Stange 37 aufweist, mittels der das Rad 35 mit dem zweiten Gleiter 4 gekuppelt ist, um den zweiten Gleiter 4 anzutreiben. Die erste Stange 36 kann an ihrem ersten Längsende mittels eines ersten Radgelenks 42 an einer Seitenfläche des Rads 35 und die zweite Stange 37 kann an ihrem zweiten Längsende mittels eines zweiten Radgelenks 43 an der Seitenfläche des Rads 35 angelenkt sein, wie es auch in Figur 6 dargestellt ist. Das erste Radgelenk 42 und das zweite Radgelenk 43 können dabei an einer Position des Rads angebracht sein, die symmetrisch bezüglich der Rotationsachse 46 des Rads 35 zueinander sind. Dadurch wird erreicht, dass entweder die erste Stange 36 unter Zug und die zweite Stange 37 unter Druck belastet werden oder die erste Stange 36 unter Druck und die zweite Stange 37 unter Zug belastet werden.

Zudem ist in Figur 6 erkennbar, dass es denkbar ist, dass die Injektoranordnung 1 eine dritte Stange 38 aufweist, die an ihrem ersten Längsende mittels eines ersten Kupplungsgelenks 44 an dem zweiten Längsende der ersten Stange 36 angelenkt ist. Das zweite Längsende der dritten Stange 38 ist mittels eines ersten Gleitergelenks 40 an dem ersten Gleiter 3 angelenkt. Zudem ist erkennbar, dass es denkbar ist, dass die Injektoranordnung 1 eine vierte Stange 39 aufweist, die an ihrem ersten Längsende mittels eines zweiten Kupplungsgelenks 45 an dem zweiten Längsende der zweiten Stange 37 angelenkt ist. Das zweite Längsende der vierten Stange 39 ist mittels eines zweiten Gleitergelenks 41 an dem zweiten Gleiter 4 angelenkt. Das erste Kupplungsgelenk 44 und/oder das zweite Kupplungsgelenk 45 können dabei eine eingeschränkte Bewegungsfreiheit haben, so dass das erste Kupplungsgelenk 44 und/oder das zweite Kupplungsgelenk 45 sich nur begrenzt von dem Injektorkörper 7 entfernen können.

Alternativ dazu, die dritte Stange 38 und die vierte Stange 39 vorzusehen, ist es denkbar, dass das zweite Längsende der ersten Stange 36 mittels eines ersten Gleitergelenks 40 an dem ersten Gleiter 3 angelenkt ist und das zweite Längsende der zweiten Stange 37 mittels eines zweiten Gleitergelenks 41 an dem zweiten Gleiter 4 angelenkt ist.

Figur 6 zeigt zudem, dass das Gelenk 13 eine Achse 33 aufweisen kann, die bei dem Verschwenken des Halters 12 rotiert und an der ein erstes Zahnrad 34 befestigt ist, und das Rad 35 ein zweites Zahnrad 35 ist, deren Zähne mit den Zähnen des ersten Zahnrads 34 in Eingriff stehen, zum Beispiel durch eine Kegelradverzahnung. Dadurch rotiert bei dem Verschwenken des Halters 12 das erste Zahnrad 34, das wiederum das zweite Zahnrad 35 antreibt. Die Rotationsachse des ersten Zahnrads 34 und die Rotationsachse der Achse 33 können zusammenfallen. Zudem können die Rotationsachse des ersten Zahnrads 34 und die Rotationsachse des zweiten Zahnrads 35 im Wesentlichen senkrecht zueinander angeordnet sein.

### Bezugszeichenliste

- 1: Injektoranordnung
- 2: Faltkeil
- 3: erster Gleiter
- 4: zweiter Gleiter
- 5: erste Gleiterbahn
- 7: Injektorkörper
- 8: Faltkammer
- 9: Intraokularlinse
- 10: Optikkörper
- 11: Haptik
- 12: Halter
- 13: Gelenk
- 14: Kanüle
- 15: erste Bewegungsrichtung
- 16: zweite Bewegungsrichtung
- 17: Adapter
- 18: Faltkammeröffnung
- 19: feststehendes Faltkeilteil
- 20: bewegliches Faltkeilteil
- 21: Gleiterzapfen
- 22: Schieber
- 23: erster Schieberzapfen
- 24: zweiter Schieberzapfen
- 25: Gleitervorsprung
- 26: erster Vorsprung
- 27: erste Gleitfläche
- 28: erster Vorsprung
- 29: zweiter Vorsprung
- 31: erste Gleitfläche
- 32: zweite Gleitfläche
- 33: Achse
- 34: erste Zahnrad
- 35: Rad, zweites Zahnrad
- 36: erste Stange
- 37: zweite Stange
- 38: dritte Stange
- 39: vierte Stange
- 40: erstes Gleitergelenk
- 41: zweites Gleitergelenk
- 42: erstes Zahnradgelenk
- 43: zweites Zahnradgelenk
- 44: erstes Kupplungsgelenk
- 45: zweites Kupplungsgelenk
- 46: Rotationsachse

## Patentansprüche

1. Injektoranordnung mit einer Faltkammer (8), welche eingerichtet ist, eine Intraokularlinse (9), die einen Optikkörper (10) und zwei Haptiken (11) aufweist, aufzunehmen, einem Halter (12) und einem Faltkeil (2), der an dem Halter (12) angebracht ist und eingerichtet ist, via eine Faltkammeröffnung (18) in die Faltkammer (8) eingebracht zu werden und dadurch die Intraokularlinse (9) zu falten, **dadurch gekennzeichnet, dass** die Injektoranordnung (1) einen ersten Gleiter (3) und einen zweiten Gleiter (4) aufweist, die jeweils eingerichtet sind, durch ein Verschieben des jeweiligen Gleiters (3, 4) aus einer Ausgangsposition in eine Endposition eine der zwei Haptiken (11) auf den Optikkörper (10) zu verschieben, wobei der Halter (12) eingerichtet ist, durch ein Bewegen des Halters (12) zu der Faltkammer (8) hin den ersten Gleiter (3) und/oder den zweiten Gleiter (4) aus der Ausgangsposition in die Endposition anzutreiben.

2. Injektoranordnung gemäß Anspruch 1, wobei die Injektoranordnung (1) einen Injektorkörper (7), in dessen Innerem die Faltkammer (8) angeordnet ist, und ein Gelenk (13) aufweist, mittels dem der Halter (12) verschwenkbar an dem Injektorkörper (7) angebracht ist.

3. Injektoranordnung gemäß Anspruch 2, wobei die Injektoranordnung (1) einen Schieber (22) aufweist, der an dem ersten Gleiter (3) befestigt ist und sich ausgehend von dem ersten Gleiter (3) in Richtung zu dem Gelenk (13) erstreckt, wobei der Halter (12) eingerichtet ist, mittels eines Verschwenkens des Halters (12) um das Gelenk (13) herum in Richtung zu der Faltkammer (8) hin, den Schieber (22) anzutreiben, der dadurch den ersten Gleiter (3) in die Endposition verschiebt.

4. Injektoranordnung gemäß Anspruch 2, wobei die Injektoranordnung einen an dem Halter (12) befestigten ersten Vorsprung (26, 28) mit einer ersten Gleitfläche (27, 31) aufweist, die eingerichtet ist, wenn der Halter (12) um das Gelenk (13) herum in Richtung zu der Faltkammer (8) hin verschwenkt wird, an dem ersten Gleiter (3) entlang zu gleiten und dadurch den ersten Gleiter (3) in die Endposition zu verschieben.

5. Injektoranordnung gemäß Anspruch 4, wobei der erste Gleiter (3) einen ersten Gleitervorsprung (25) aufweist, der von einer dem zweiten Gleiter (4) abgewandten Seite des ersten Gleiters (3) vorsteht und an dem die erste Gleitfläche (27, 31) eingerichtet ist zu gleiten, und/oder wobei der zweite Gleiter (4) einen zweiten Gleitervorsprung aufweist, der von einer dem ersten Gleiter (3) abgewandten Seite des zweiten Gleiters (4) vorsteht und an dem die zweite Gleitfläche (32) eingerichtet ist zu gleiten.

6. Injektoranordnung gemäß Anspruch 2, wobei die Injektoranordnung (1) ein Rad aufweist, das eingerichtet ist, durch eine Rotation des Rads den ersten Gleiter (3) von der Ausgangsposition in die Endposition zu verschieben, und der Halter (12) eingerichtet ist, mittels eines Verschwenkens des Halters (12) um das Gelenk (13) herum in Richtung zu der Faltkammer (8) hin das Rad in die Rotation zu versetzen.

7. Injektoranordnung gemäß Anspruch 6, wobei das Rad eingerichtet ist, durch die Rotation den zweiten Gleiter (4) von der Ausgangsposition in die Endposition zu verschieben.

8. Injektoranordnung gemäß Anspruch 6 oder 7, wobei die Injektoranordnung (1) eine erste Stange (36) aufweist, mittels der das Rad mit dem ersten Gleiter (3) gekuppelt ist, um den ersten Gleiter (3) anzutreiben, und/oder eine zweite Stange (37) aufweist, mittels der das Rad mit dem zweiten Gleiter (4) gekuppelt ist, um den zweiten Gleiter (4) anzutreiben.

9. Injektoranordnung gemäß einem der Ansprüche 6 bis 8, wobei das Gelenk (13) eine Achse (33) aufweist, die bei dem Verschwenken des Halters (12) rotiert und an der ein erstes Zahnrad (34) befestigt ist, und das Rad ein zweites Zahnrad (35) ist, deren Zähne mit den Zähnen des ersten Zahnrads (34) in Eingriff stehen.

10. Ophthalmochirurgischer Injektor mit einer Injektoranordnung gemäß einem der Ansprüche 1 bis 9.

## Claims

1. Injector assembly having a folding chamber (8) which is designed to accommodate an intraocular lens (9) comprising an optics body (10) and two haptics (11), a holder (12) and a folding wedge (2), which is attached to the holder (12) and is designed to be introduced into the folding chamber (8) via a folding chamber opening (18) and thereby to fold the intraocular lens (9), **characterized in that** the injector assembly (1) has a first slider (3) and a second slider (4) which are each designed to displace one of the two haptics (11) onto the optics body (10) by displacement of the respective slider (3, 4) from a starting position into an end position, wherein the holder (12) is designed to drive the first slider (3) and/or the second slider (4) from the starting position into the end position by movement of the holder (12) toward the folding chamber (8).

2. Injector assembly according to Claim 1, wherein the injector assembly (1) has an injector body (7), inside which the folding chamber (8) is arranged, and a joint (13) by means of which the holder (12) is attached pivotably to the injector body (7).

3. Injector assembly according to Claim 2, wherein the injector assembly (1) has a pusher (22) which is fastened to the first slider (3) and extends from the first slider (3) toward the joint (13), wherein the holder (12) is designed to drive the pusher (22), by means of pivoting of the holder (12) about the joint (13) in the direction of the folding chamber (8), which thereby displaces the first slider (3) into the end position.

4. Injector assembly according to Claim 2, wherein the injector assembly has a first protrusion (26, 28) which is fastened to the holder (12) and has a first sliding surface (27, 31) which is designed to slide along the first slider (3), when the holder (12) is pivoted about the joint (13) in the direction of the folding chamber (8), and thereby to displace the first slider (3) into the end position.

5. Injector assembly according to Claim 4, wherein the first slider (3) has a first slider protrusion (25) which protrudes from a side of the first slider (3) facing away from the second slider (4) and on which the first sliding surface (27, 31) is designed to slide, and/or wherein the second slider (4) has a second slider protrusion which protrudes from a side of the second slider (4) facing away from the first slider (3) and on which the second sliding surface (32) is designed to slide.

6. Injector assembly according to Claim 2, wherein the injector assembly (1) has a wheel which is designed to displace the first slider (3) from the starting position into the end position by rotation of the wheel, and the holder (12) is designed to set the wheel in rotation by means of pivoting the holder (12) about the joint (13) toward the folding chamber (8).

7. Injector assembly according to Claim 6, wherein the wheel is designed to displace the second slider (4) from the starting position into the end position as a result of the rotation.

8. Injector assembly according to Claim 6 or 7, wherein the injector assembly (1) has a first rod (36), by means of which the wheel is coupled to the first slider (3) to drive the first slider (3), and/or has a second rod (37), by means of which the wheel is coupled to the second slider (4) to drive the second slider (4).

9. Injector assembly according to one of Claims 6 to 8, wherein the joint (13) has a pin (33) which rotates upon pivoting of the holder (12) and to which a first gearwheel (34) is fastened, and the wheel is a second gearwheel (35), the teeth of which mesh with the teeth of the first gearwheel (34).

10. Ophthalmosurgical injector having an injector assembly according to one of Claims 1 to 9.

## Revendications

1. Agencement d'injecteur comprenant une chambre de pliage (8) adaptée pour recevoir une lentille intraoculaire (9) présentant un corps optique (10) et deux éléments haptiques (11), un support (12) et un coin de pliage (2) monté sur le support (12) et adapté pour être introduit dans la chambre de pliage (8) via une ouverture de chambre de pliage (18) et pour plier ainsi la lentille intraoculaire (9), **caractérisé en ce que** l'agencement d'injecteur (1) présente un premier coulisseau (3) et un deuxième coulisseau (4) qui sont chacun adaptés pour déplacer l'un des deux éléments haptiques (11) sur le corps optique (10) par un déplacement du coulisseau respectif (3, 4) d'une position initiale à une position finale, le support (12) étant adapté pour entraîner le premier coulisseau (3) et/ou le deuxième coulisseau (4) de la position initiale à la position finale par un mouvement du support (12) vers la chambre de pliage (8).

2. Agencement d'injecteur selon la revendication 1, l'agencement d'injecteur (1) présentant un corps d'injecteur (7), à l'intérieur duquel est agencée la chambre de pliage (8), et une articulation (13) au moyen de laquelle le support (12) est monté pivotant sur le corps d'injecteur (7).

3. Agencement d'injecteur selon la revendication 2, l'agencement d'injecteur (1) présentant un poussoir (22) fixé au premier coulisseau (3) et s'étendant depuis le premier coulisseau (3) en direction de l'articulation (13), le support (12) étant adapté pour entraîner, au moyen d'un pivotement du support (12) autour de l'articulation (13) en direction de la chambre de pliage (8), le poussoir (22) qui déplace ainsi le premier coulisseau (3) vers la position finale.

4. Agencement d'injecteur selon la revendication 2, l'agencement d'injecteur présentant une première saillie (26, 28) fixée au support (12), comprenant une première surface de coulissement (27, 31) qui, lorsque le support (12) est pivoté autour de l'articulation (13) en direction de la chambre de pliage (8), est adaptée pour coulisser le long du premier coulisseau (3) et déplacer ainsi le premier coulisseau (3) vers la position finale.

5. Agencement d'injecteur selon la revendication 4, dans lequel le premier coulisseau (3) présente une première saillie de coulisseau (25) qui fait saillie depuis un côté du premier coulisseau (3) détourné du deuxième coulisseau (4) et sur laquelle la première surface de coulissement (27, 31) est adaptée pour coulisser, et/ou dans lequel le deuxième coulisseau (4) présente une deuxième saillie de coulisseau qui fait saillie depuis un côté du deuxième coulisseau (4) détourné du premier coulisseau (3) et sur laquelle la deuxième surface de coulissement (32) est adaptée pour coulisser.

6. Agencement d'injecteur selon la revendication 2, l'agencement d'injecteur (1) présentant une roue adaptée pour déplacer le premier coulisseau (3) de la position initiale à la position finale par une rotation de la roue, et le support (12) étant adapté pour mettre la roue en rotation au moyen d'un pivotement du support (12) autour de l'articulation (13) en direction de la chambre de pliage (8).

7. Agencement d'injecteur selon la revendication 6, dans lequel la roue est adaptée pour déplacer le deuxième coulisseau (4) de la position initiale à la position finale par la rotation.

8. Agencement d'injecteur selon la revendication 6 ou 7, l'agencement d'injecteur (1) présentant une première tige (36) au moyen de laquelle la roue est couplée au premier coulisseau (3) afin d'entraîner le premier coulisseau (3), et/ou une deuxième tige (37) au moyen de laquelle la roue est couplée au deuxième coulisseau (4) afin d'entraîner le deuxième coulisseau (4).

9. Agencement d'injecteur selon l'une quelconque des revendications 6 à 8, dans lequel l'articulation (13) présente un axe (33) qui tourne lors du pivotement du support (12) et auquel est fixée une première roue dentée (34), et la roue est une deuxième roue dentée (35) dont les dents sont en prise avec les dents de la première roue dentée (34).

10. Injecteur ophtalmochirurgical comprenant un agencement d'injecteur selon l'une quelconque des revendications 1 à 9.
